# EUROPEAN PATENT APPLICATION

(11) **EP 4 400 103 A1**
(43) Date of publication of application: **17.07.2024**
(21) Application number: 22867409.9
(22) Date of filing: 08.09.2022
(51) Int. Cl.: A61K 31/385, A61K 38/06, A61P 21/00, A61P 43/00

(54) **METHOD FOR PREVENTING NITRATION OF TYROSINE RESIDUES IN HEPATOCYTE GROWTH FACTOR USING TRISULFIDE COMPOUND**

(30) Priority: 09.09.2021 JP 2021146753
(71) Applicant: Kyushu University, National University Corporation, Nishi-ku Fukuoka-shi Fukuoka 819-0395 (JP); Kyowa Hakko Bio Co., Ltd., Tokyo 100-8185 (JP)
(72) Inventor: TATSUMI Ryuichi, Fukuoka-shi, Fukuoka 819-0395 (JP); NAKAMURA Mako, Fukuoka-shi, Fukuoka 819-0395 (JP); OHSHIMA Etsuo, Tokyo 100-8185 (JP)
(74) Representative: Vossius & Partner Patentanwälte Rechtsanwälte mbB
(86) International application number: PCT/JP2022/033764
(87) International publication number: WO 2023/038088

(57) **Abstract**

The present invention provides a trisulfide-compound-containing agent for inhibiting nitration of tyrosine residues in hepatocyte growth factor, the trisulfide compound being: glutathione trisulfide or a pharmaceutically acceptable salt thereof; or a compound represented by formula (1) (wherein X represents -OR¹ or -NR²R³, R¹ represents a hydrogen atom or an alkyl group containing 1 to 6 carbon atoms, R² and R³ independently represent a hydrogen atom or an alkyl group containing 1 to 6 carbon atoms, and the alkyl group may have one or more substituents selected from the group consisting of amino groups and carboxy groups), a pharmaceutically acceptable salt thereof, or a cyclodextrin clathrate thereof.

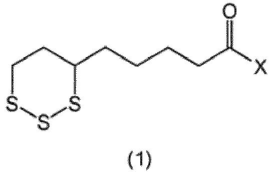

## Description

### Technical Field

The present invention relates to a method for preventing nitration of tyrosine residues in hepatocyte growth factor using trisulfide compounds.

### Background Art

Hepatocyte Growth Factor (hereinafter also referred to as "HGF") was discovered as an endogenous protein that promotes the proliferation of mature hepatocytes, but subsequent studies have revealed that HGF has various physiological activities such as promotion of cell movement, inhibition of cell death, induction of morphogenesis, anti-fibrosis and angiogenesis in addition to cell proliferation, and plays a role in regeneration and protection of not only liver but also various tissues and organs such as gastrointestinal mucosa, nerve, lung, kidney, heart and skin (Non-Patent Literatures 5 to 7).

HGF has been further reported to be an activator of muscle stem cells (satellite cells), and the satellite cells are known to contribute to regeneration of muscle fibers and the like (Non-Patent Document 1).

In recent years, it has been reported that when tyrosine residues in the protein of HGF are nitrated, the physiological activities of HGF disappear (Non-Patent Literatures 2 and 8) (Ryuichi Tatsumi, "Fundamental Science of Muscle Stem Cells and Their Applications in Health and Medicine", "Kyushu University", Industry Academia Government Collaboration Promoting Seed Presentation, in Tokyo 2019, March 14, 2019) (A. Elgaabari et al., "Insight linking between nitration and myogenic dysfunction of HGF/NK1 domain", The 128th Annual Meeting of Japanese Society of Animal Science). In these reports, it is suggested that the dysfunction of HGF causes inhibition of activation of the satellite cells and decrease in proliferation of the satellite cell. Accordingly, it is considered that the nitration of tyrosine residues in the protein of HGF inhibits muscle growth, hypertrophy, regeneration or maintenance, resulting in muscle atrophy, muscle regeneration failure, inhibition of muscle growth and the like.

Trisulfide compounds such as glutathione trisulfide are converted *in vivo* to active sulfur species such as glutathione persulfide. It has been reported that the active sulfur species have a strong anti-oxidant action and may have physiological functions such as anti-aging (for example, Non-Patent Literatures 3 and 4). Further, as a method for producing trisulfide compounds, a method described in Patent Literature 1 and the like are known.

### Citation List

### Patent Literature

[Patent Literature 1] WO 2018/117186

### Non-Patent Literature

[Non-Patent Literature 1] R. Tatsumi, "Mechano-biology of skeletal muscle hypertrophy and regeneration: possible mechanism of stretch-induced activation of resident myogenic stem cells", Animal Science Journal 81(1), 11-20 (2010).
[Non-Patent Literature 2] Nana Imatomi et al., "Physiologic significance of nitrated/dysfunction of myogenic stem satellite cell activator HGF: breakthrough of major factors for age-related muscle atrophy with impaired regeneration", The 128th Annual Meeting of Japanese Society of Animal Science, abstract
[Non-Patent Literature 3] T. Ida, et al., "Reactive cysteine persulfides and S-polythiolation regulate oxidative stress and redox signaling", PNAS, May 27, 2014, 111(21) 7606-7611
[Non-Patent Literature 4] T. Akaike, et al., "Cysteinyl-tRNA synthetase governs cysteine polysulfidation and mitochondrial bioenergetics", Nature Communications, 2017, Oct 27; 8(1): 1177
[Non-Patent Literature 5] Hirohito Tsubouchi, "Discovery and clinical application of hepatocyte growth factor", Journal of Japanese Society of Gastroenterology, Vol. 110, No. 12, pp. 2033 to 2041 (2013)
[Non-Patent Literature 6] Shinya Mizuno and Toshikazu Nakamura, "Clinical application of HGF, a tissue repair factor", Drug Delivery System, 16-6 (2001).
[Non-Patent Literature 7] C. Desole, et al., "HGF and MET: From Brain Development to Neurological Disorders", Frontiers in Cell and Developmental Biology, Vol. 9 (published 09 June 2021, Article 683609).
[Non-Patent Literature 8] A. Elgaabari, et. al., "A pilot study on nitration/dysfunction of NK1 segment of myogenic stem cell activator HGF", Biochemistry and Biophysics Reports, Vol. 31, 101295 (2022) (published June 11, 2022)

### Summary of Invention

### Technical Problem

An object of the present invention is to provide a nitration inhibitor of a tyrosine residue in HGF.

### Solution to Problem

The present inventors have found that only HGF among cell growth factors undergoes nitration of tyrosine residues, and considered that the nitration of tyrosine residues in HGF has important physiological significance. Then, the present inventors have found that glutathione trisulfide and lipoic acid trisulfide (a compound represented by the formula (4) below) inhibit nitration of tyrosine residues in HGF, which is considered to be important as described above, in a concentration-dependent manner, and have completed the present invention.

That is, the present invention provides the following [1] to [30].
[1] A nitration inhibitor of a tyrosine residue in hepatocyte growth factor, comprising a trisulfide compound,
   wherein the trisulfide compound is glutathione trisulfide or a pharmaceutically acceptable salt thereof or a compound of formula (1): 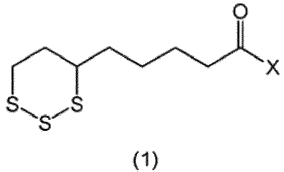 wherein X represents -OR¹ or -NR²R³, R¹ represents a hydrogen atom or an alkyl group having 1 to 6 carbon atoms, R² and R³ each independently represent a hydrogen atom or an alkyl group having 1 to 6 carbon atoms, and the alkyl group optionally has one or more substituents selected from the group consisting of an amino group and a carboxy group (hereinafter, also referred to as "Compound (1)"), or a pharmaceutically acceptable salt thereof or a cyclodextrin clathrate thereof.
[2] The nitration inhibitor according to [1], wherein the trisulfide compound is glutathione trisulfide or a pharmaceutically acceptable salt thereof.
[3] The nitration inhibitor according to [2], wherein glutathione trisulfide or the pharmaceutically acceptable salt thereof includes at least one selected from the group consisting of glutathione trisulfide, an amino acid salt of glutathione trisulfide, and an alkali metal salt of glutathione trisulfide.
[4] The nitration inhibitor according to [2], wherein glutathione trisulfide or the pharmaceutically acceptable salt thereof includes at least one selected from the group consisting of glutathione trisulfide, an arginine salt of glutathione trisulfide, and a sodium salt of glutathione trisulfide.
[5] The nitration inhibitor according to [1], wherein the trisulfide compound is lipoic acid trisulfide or a pharmaceutically acceptable salt thereof.
[6] A preventive or therapeutic agent for muscle atrophy and/or muscle regeneration failure, comprising the nitration inhibitor according to any one of [1] to [5].
[7] A method for preventing or treating muscle atrophy and/or muscle regeneration failure, comprising administering the nitration inhibitor according to any one of [1] to [5] to a subject in need thereof.
[8] A method for preventing or treating muscle atrophy and/or muscle regeneration failure in a companion animal, comprising administering the nitration inhibitor according to any one of [1] to [5] to the companion animal.
[9] The nitration inhibitor according to any one of [1] to [5] for use in preventing or treating muscle atrophy and/or muscle regeneration failure.
[10] Use of the nitration inhibitor according to any one of [1] to [5] for the manufacture of a preventive or therapeutic agent for muscle atrophy and/or muscle regeneration failure.
[11] An agent for suppressing or improving inhibition of muscle growth of livestock or poultry (decrease in meat productivity) caused by heat stress, comprising the nitration inhibitor according to any one of [1] to [5].
[12] A method for suppressing or improving inhibition of muscle growth (decrease in meat productivity) caused by heat stress, comprising administering the nitration inhibitor according to any one of [1] to [5] to livestock or the poultry in need thereof.
[13] A method for suppressing or improving inhibition of muscle growth of livestock or poultry (decrease in meat productivity) caused by heat stress, comprising administering the nitration inhibitor according to any one of [1] to [5] to the livestock or the poultry.
[14] The nitration inhibitor according to any one of [1] to [5] for use in suppressing or improving inhibition of muscle growth of livestock or poultry (decrease in meat productivity) caused by heat stress.
[15] Use of the nitration inhibitor according to any one of [1] to [5] for the manufacture of an agent for suppressing or improving inhibition of muscle growth of livestock or poultry (decrease in meat productivity) caused by heat stress.
[16] A method for inhibiting nitration of a tyrosine residue in hepatocyte growth factor, comprising administering a trisulfide compound to a subject in need thereof, wherein the trisulfide compound is glutathione trisulfide or a pharmaceutically acceptable salt thereof, or Compound (1), a pharmaceutically acceptable salt thereof, a cyclodextrin clathrate thereof, a pharmaceutically acceptable salt thereof, or a cyclodextrin clathrate thereof.
[17] The method according to [16], wherein the trisulfide compound is glutathione trisulfide or a pharmaceutically acceptable salt thereof.
[18] The method according to [17], wherein glutathione trisulfide or the pharmaceutically acceptable salt thereof includes at least one selected from the group consisting of glutathione trisulfide, an amino acid salt of glutathione trisulfide, and an alkali metal salt of glutathione trisulfide.
[19] The method according to [17], wherein glutathione trisulfide or the pharmaceutically acceptable salt thereof includes at least one selected from the group consisting of glutathione trisulfide, an arginine salt of glutathione trisulfide, and a sodium salt of glutathione trisulfide.
[20] The method according to [16], wherein the trisulfide compound is lipoic acid trisulfide or a pharmaceutically acceptable salt thereof.
[21] A trisulfide compound for use in inhibiting nitration of a tyrosine residue in hepatocyte growth factor, wherein the trisulfide compound is glutathione trisulfide or a pharmaceutically acceptable salt thereof, or Compound (1), a pharmaceutically acceptable salt thereof, a cyclodextrin clathrate thereof, a pharmaceutically acceptable salt thereof, or a cyclodextrin clathrate thereof.
[22] The trisulfide compound for use according to [21], wherein the trisulfide compound is glutathione trisulfide or a pharmaceutically acceptable salt thereof.
[23] The trisulfide compound for use according to [22], wherein glutathione trisulfide or the pharmaceutically acceptable salt thereof includes at least one selected from the group consisting of glutathione trisulfide, an amino acid salt of glutathione trisulfide, and an alkali metal salt of glutathione trisulfide.
[24] The trisulfide compound for use according to [22], wherein glutathione trisulfide or the pharmaceutically acceptable salt thereof includes at least one selected from the group consisting of glutathione trisulfide, an arginine salt of glutathione trisulfide, and a sodium salt of glutathione trisulfide.
[25] The trisulfide compound for use according to [21], wherein the trisulfide compound is lipoic acid trisulfide or a pharmaceutically acceptable salt thereof.
[26] Use of a trisulfide compound for the manufacture of a nitration inhibitor of a tyrosine residue in hepatocyte growth factor, wherein the trisulfide compound is glutathione trisulfide or a pharmaceutically acceptable salt thereof, or Compound (1), a pharmaceutically acceptable salt thereof, a cyclodextrin clathrate thereof, a pharmaceutically acceptable salt thereof, or a cyclodextrin clathrate thereof.
[27] The use according to [26], wherein the trisulfide compound is glutathione trisulfide or a pharmaceutically acceptable salt thereof.
[28] The use according to [27], wherein glutathione trisulfide or the pharmaceutically acceptable salt thereof includes at least one selected from the group consisting of glutathione trisulfide, an amino acid salt of glutathione trisulfide, and an alkali metal salt of glutathione trisulfide.
[29] The use according to [27], wherein glutathione trisulfide or the pharmaceutically acceptable salt thereof includes at least one selected from the group consisting of glutathione trisulfide, an arginine salt of glutathione trisulfide, and a sodium salt of glutathione trisulfide.
[30] The use according to [26], wherein the trisulfide compound is lipoic acid trisulfide or a pharmaceutically acceptable salt thereof.

### Advantageous Effects of Invention

According to the present invention, it is possible to provide a nitration inhibitor of a tyrosine residue in HGF.

### Brief Description of Drawings

Fig. 1 shows the results of Western blot analysis on the inhibitory effect of glutathione trisulfide on nitration of recombinant HGF.
Fig. 2 shows the results of Western blot analysis on the inhibitory effect of lipoic acid trisulfide on nitration of recombinant HGF.

### Description of Embodiments

The contents of the present invention will be described in detail, but the present invention is not limited to the following embodiments. In the present specification, "inhibition of muscle growth and/or decrease in meat productivity" may be referred to as "inhibition of muscle growth (decrease in meat productivity)".

The nitration inhibitor, the preventing or treating agent for muscle atrophy and/or muscle regeneration failure, and the method for preventing or treating of the present invention can be administered or applied to a human or a companion animal. The nitration inhibitor, the agent for suppressing or improving inhibition of muscle growth (decrease in meat productivity) caused by heat stress, and the method for suppressing or improving inhibition of muscle growth of the present invention can be administered or applied to livestock or poultry.

In human HGF, the tyrosine residues at positions 198 and 250 can be nitrated. Among companion animals and livestock described later, in mammals HGF, the tyrosine residues corresponding to the tyrosine residues at positions 198 and 250 of human HGF may be nitrated. Among companion animals, livestock, and poultry described below, in avian HGF, the tyrosine residue corresponding to tyrosine residues at position 250 of human HGF may be nitrated. The nitration of the tyrosine residues at positions 198 and/or 250 in human HGF and the tyrosine residues in non-human-derived HGF at positions corresponding thereto deactivates HGF brings disappearance of HGF activities. Dysfunction of HGF leads to inhibition of growth, hypertrophy, regeneration or maintenance of muscle, and is considered to cause muscle atrophy, muscle regeneration failure, and the like. The nitration inhibitor of the present invention can inhibit the nitration of the tyrosine residues at the above-mentioned specific positions.

The trisulfide compound used in the nitration inhibitor of the present invention is glutathione trisulfide or a pharmaceutically acceptable salt thereof, or Compound (1), a pharmaceutically acceptable salt thereof, or a cyclodextrin clathrate thereof. Glutathione trisulfide is represented by the formula (2):

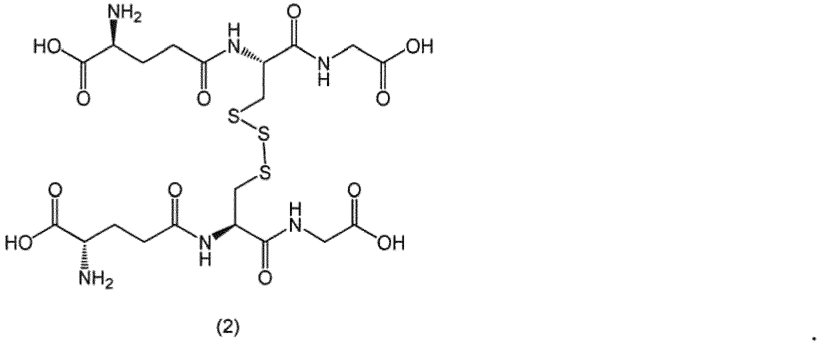

In the present invention, examples of the pharmaceutically acceptable salts include salts with inorganic acids such as hydrochloric acid, hydrobromic acid, sulfuric acid, nitric acid and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid and p-toluenesulfonic acid; salts with alkali metals such as sodium and potassium; salts with alkaline earth metals such as calcium and magnesium; ammonium salts; and salts with amino acids such as arginine.

The pharmaceutically acceptable salt of glutathione trisulfide is preferably an amino acid salt or an alkali metal salt, and more preferably an arginine salt or a sodium salt. The pharmaceutically acceptable salt of Compound (1) is preferably a salt with an alkali metal, and more preferably a sodium salt.

Glutathione trisulfide or a pharmaceutically acceptable salt thereof or Compound (1) or a pharmaceutically acceptable salt thereof may show crystal polymorphism, but is not limited to any crystal form, and may be a single substance or a mixture of any crystal forms. In addition, glutathione trisulfide or a pharmaceutically acceptable salt thereof or Compound (1) or a pharmaceutically acceptable salt thereof also includes an amorphous form. Glutathione trisulfide or a pharmaceutically acceptable salt thereof or Compound (1) or a pharmaceutically acceptable salt thereof includes an anhydrate and a solvate (in particular, a hydrate).

Compound (1), in one embodiment, is a compound represented by the formula (3): 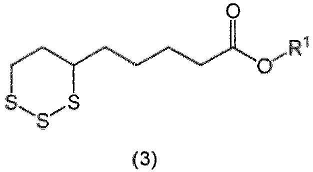 wherein R¹ represents a hydrogen atom or an alkyl group having 1 to 6 carbon atoms, and R¹ may be, for example, a hydrogen atom, methyl, ethyl, propyl, butyl, pentyl or hexyl. Preferably, R¹ is a hydrogen atom, and in this case, the compound represented by the formula (3) is lipoic acid trisulfide represented by the formula (4):

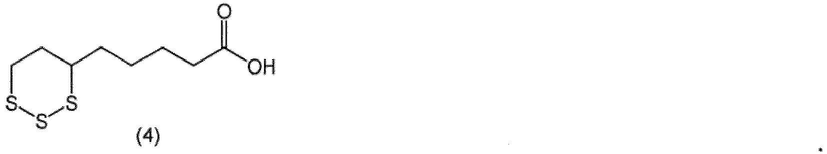

Compound (1) may, in another embodiment, be represented by the formula (5): 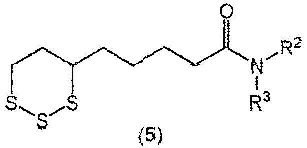 wherein R² and R³ each independently represent a hydrogen atom or an alkyl group having 1 to 6 carbon atoms, and the alkyl group optionally has one or more substituents selected from the group consisting of an amino group and a carboxy group. R² and R³ may be a hydrogen atom, or an alkyl group such as methyl, ethyl, propyl, butyl, pentyl and hexyl. These alkyl groups optionally have substituents of one or both of an amino group and a carboxy group. R² and R³ may be, for example, a group represented by the formula (6) (in the formula, * represents a bond.). Specific examples of the compound represented by the formula (5) include a compound in which both R² and R³ are hydrogen atoms, and a compound in which R² is a hydrogen atom and R³ is a group represented by the formula (6).

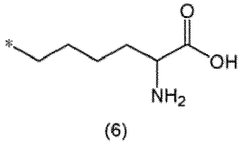

The compound represented by the formula (5) can be produced by a step of oxidizing the compound represented by the formula (5a) with an oxidizing agent to give a sulfoxide compound (step 1) and a step of allowing the obtained sulfoxide compound to react with a sulfur source (step 2). 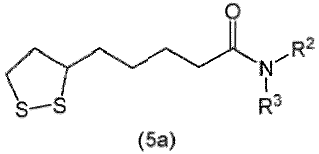 wherein R² and R³ each independently represent a hydrogen atom or an alkyl group having 1 to 6 carbon atoms, and the alkyl group optionally has one or more substituents selected from the group consisting of an amino group and a carboxy group.

In the above-described production method, Step 1 and Step 2 may be performed in a one-pot reaction without isolating the sulfoxide compound.

A solvent used in Step 1 is not particularly limited as long as it dissolves the compound represented by the formula (5a) and an oxidizing agent and does not inhibit the oxidation reaction. Examples of such solvents include water, a sulfuric acid aqueous solution, an ethanol aqueous solution, and an acetonitrile aqueous solution, and water is preferable. The amount of solvent used in Step 1 can be1 mL to 500 mL, preferably 10 mL to 20 mL, with respect to 1 gram of the compound represented by the formula (5a).

Examples of oxidizing agents used in Step 1 include potassium peroxymonosulfate (which has been sold under a trade name such as Oxone^{®} or the like), peracetic acid, hydrogen peroxide, and sodium periodate. Hydrogen peroxide may be used with a catalytic amount of methyltrioxorhenium. Potassium peroxymonosulfate is a preferred oxidizing agent from the viewpoints of safety and costs. The amount of oxidizing agent used can be 0.8 equivalents to 2.0 equivalents, preferably 1.0 equivalent to 1.3 equivalents, with respect to 1 equivalent of the compound represented by the formula (5a).

The reaction temperature in Step 1 can be -20°C to 30°C, preferably -5°C to 5°C.

The reaction time of Step 1 can be 5 minutes to 24 hours, preferably 0.5 hours to 2 hours.

A solvent used in Step 2 is not particularly limited as long as it dissolves a sulfoxide compound and a sulfur source and does not inhibit the reaction thereafter. Examples of such solvents include water, a sulfuric acid aqueous solution, an ethanol aqueous solution, and an acetonitrile aqueous solution, and water is preferable. The amount of solvent used in Step 2 can be 1 mL to 500 mL, preferably 10 mL to 20 mL, with respect to 1 gram of the sulfoxide compound.

Examples of sulfur sources used in Step 2 include sodium sulfide, potassium sulfide, sodium hydrosulfide, potassium hydrosulfide, and hydrogen sulfide. The amount of sulfur source used can be 0.5 equivalents to 4.0 equivalents, preferably 0.9 equivalents to 1.2 equivalents, with respect to 1 equivalent of the sulfoxide compound.

The reaction temperature in Step 2 can be -20°C to 30°C, preferably -5°C to 25°C.

The reaction time of Step 2 can be 10 minutes to 2 days, preferably 0.5 hours to 2 hours.

In a case where Step 1 and Step 2 are performed in a one-pot reaction, examples of reaction solvents include water, a sulfuric acid aqueous solution, an ethanol aqueous solution, and an acetonitrile aqueous solution, and water is preferable, and the amount of solvent can be 1 mL to 500 mL, preferably 10 mL to 20 mL, with respect to 1 gram of the compound represented by the formula (5a). Examples of oxidizing agents used include potassium peroxymonosulfate, peracetic acid, hydrogen peroxide (which may be used with a catalytic amount of metilyltrioxorhenium), and sodium periodate, preferably potassium peroxymonosulfate. The amount of oxidizing agent used can be 0.8 equivalents to 2.0 equivalents, preferably 1.0 equivalent to 1.3 equivalents, with respect to 1 equivalent of the compound represented by the formula (5a). Examples of sulfur sources used include sodium sulfide, potassium sulfide, sodium hydrosulfide, potassium hydrosulfide, and hydrogen sulfide. The amount of sulfur source used can be 0.5 equivalents to 4.0 equivalents, preferably 0.9 equivalents to 1.2 equivalents, with respect to 1 equivalent of the compound represented by the formula (5a). The reaction temperature can be -20°C to 30°C, preferably -5°C to 25°C. The reaction time can be 15 minutes to 2 days, preferably 1 hour to 4 hours.

In addition to Step 1 and Step 2, a step of protecting functional groups such as a hydroxy group, a carbonyl group, an amino group, and a carboxy group and a step of deprotecting the protected functional groups may be comprised as necessary. Protective groups for these functional groups and protection and deprotection reactions are well known to those skilled in the art, and appropriate protective groups and protection and deprotection reactions can be selected with reference to "Greene's Protective Groups in Organic Synthesis" and the like.

The compound represented by the formula (5a) can be produced by condensing lipoic acid and NHR²R³. Examples of solvents for a condensation reaction include dichloromethane, chloroform, and tetrahydrofuran, and tetrahydrofuran is preferable. The amount of solvent can be 1 mL to 200 mL, preferably 3 mL to 35 mL, with respect to 1 gram of the compound represented by the formula (5a). Examples of condensing agents used include 1-(3-dimethylaminopropyl)-3-ethylcarbodiimide (EDC) and a salt thereof, N,N'-dicyclohexylcarbodiimide (DCC), diisopropylcarbodiimide (DIC) (in which N-hydroxysuccinimide (NHS) and 1-hydroxybenzotriazole (HOBt) may be used as additives), 4-dimethylaminopyridine (DMAP), and 1,1'-carbonyl diimidazole di(1H-imidazole-1-yl)methanone (CDI). The amount of condensing agent used can be 0.8 equivalents to 2.0 equivalents, preferably 1.0 equivalent to 1.5 equivalents, with respect to 1 equivalent of the compound represented by the formula (5a), The reaction temperature can be -10°C to 40°C, preferably 15°C to 25°C. The reaction time can be 1 hour to 3 days, preferably 1 hour to 24 hours.

The compound represented by the formula (5) can also be produced by condensing lipoic acid trisulfide and NHR²R³. The conditions of condensation are the same as above.

The compound represented by the formula (3) in which R¹ is an alkyl group having 1 to 6 carbon atoms can be produced by a step of oxidizing the compound represented by the formula (3a) with an oxidizing agent to give a sulfoxide compound (step 1) and a step of allowing the obtained sulfoxide compound to react with a sulfur source (step 2). The reaction conditions are the same as described above. 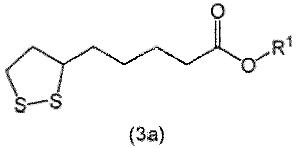 wherein R¹ represents a hydrogen atom or an alkyl group having 1 to 6 carbon atoms.

The compound represented by the formula (3a) can be produced by condensing lipoic acid and R¹OH. This is the same as described above.

The compound represented by the formula (3) in which R¹ is an alkyl group having 1 to 6 carbon atoms can also be produced by condensing lipoic acid trisulfide and R¹OH. The conditions of the condensation are the same as described above.

Cyclodextrins may be α-cyclodextrin, β-cyclodextrin, γ-cyclodextrin, or derivatives thereof. Here, "derivatives" mean that a hydrogen atom of at least one hydroxyl group in each cyclodextrin is substituted with a saccharide or an alkyl group which may have a substituent. As the cyclodextrin derivatives, it is possible to use, for example, methyl-α-cyclodextrin, methyl-β-cyclodextrin, methyl-γ-cyclodextrin, dimethyl-α-cyclodextrin, dimethyl-β-cyclodextrin, dimethyl-γ-cyclodextrin, hydroxyethyl-α-cyclodextrin, hydroxyethyl-β-cyclodextrin, hydroxyethyl-γ-cyclodextrin, 2-hydroxypropyl-α-cyclodextrin, 2-hydroxypropyl-β-cyclodextrin, 2-hydroxypropyl-γ-cyclodextrin, glucosyl-α-cyclodextrin, glucosyl-β-cyclodextrin, glucosyl-γ-cyclodextrin, maltosyl-α-cyclodextrin, maltosyl-β-cyclodextrin, maltosyl-γ-cyclodextrin, sulfobutyl ether-α-cyclodextrin, sulfobutyl ether-β-cyclodextrin, and sulfobutyl ether-γ-cyclodextrin.

A cyclodextrin clathrate can be produced through a step of dissolving a cyclodextrin in a solvent (Step a), a step of charging Compound (1) or a pharmaceutically acceptable salt thereof to the obtained solution and stirring the mixture (Step b), and a step of filtering the stirred solution, washing the solvent used in Step a with the same solvent, and freezing the filtrate to perform freeze-drying (Step c). The filtration and washing operation in Step c may be omitted.

The solvent used in Step a is preferably water.

The amount of solvent used in Step a can be 1 to 350 mL, preferably 1 to 80 mL, with respect to 1 gram of the cyclodextrin.

In Step b, the mass ratio of the cyclodextrin to Compound (1) or a pharmaceutically acceptable salt thereof can be 2 to 20, preferably 5 to 16.5.

The stirring temperature in Step b can be 20°C to 50°C, and may be room temperature.

The stirring time in Step b can be 0.25 to 40 hours, preferably 2 to 35 hours.

In Step b, after charging Compound (1) or a pharmaceutically acceptable salt thereof, the same solvent as that used in Step a may be added before stirring. The amount of solvent at this time can be 0 to 30 mL, preferably a to 20 mL, with respect to 1 gram of the cyclodextrin.

The amount of solvent used in Step c can be 0 to 150 mL, preferably 0 to 20 mL, with respect to 1 gram of the cyclodextrin.

The freezing temperature in Step c can be -30°C to -20°C, preferably -20°C.

The freezing time in Step c can be 10 to 50 hours.

The freeze-drying in Step c can be performed at an absolute pressure of 20 to 100 Pa and an external temperature of 10°C to 40°C, preferably 20°C.

The freeze-drying time in Step c can be 1 to 5 days.

A preventive or therapeutic agent for muscle atrophy and/or muscle regeneration failure of the present invention comprises the nitration inhibitor.

Examples of the muscular atrophy in the present invention include those resulting from a state in which muscles are not used for a long time or a state in which the use frequency or strength is reduced due to aging, bedridden, trauma, post-operative fixation, weightlessness, suffering from some disease, or the like. The muscular atrophy or muscular regeneration failure in the present invention relates to a disorder of growth, hypertrophy, regeneration or maintenance of muscles, which is initiated by inhibition of activation or decrease in proliferation of muscle stem cells (satellite cells), and representative examples thereof include age-related sarcopenia, amyotrophic lateral sclerosis, spinal muscular atrophy, spinal bulbar muscular atrophy and muscular dystrophy.

Examples of companion animals include dogs, cats, hamsters, rabbits, ferrets, parakeets, Java sparrow, parrots, and the like.

The dose of the nitration inhibitor and the preventive or therapeutic agent for muscle atrophy and muscle regeneration failure of the present invention is a therapeutically effective amount and varies depending on symptoms, age, administration route, dosage form, and the like. In typical cases, glutathione trisulfide or a pharmaceutically acceptable salt thereof, or Compound (1), a pharmaceutically acceptable salt thereof, or a cyclodextrin clathrate thereof can be administered to an adult at 0.5 to 2000 mg per day, and the administration of 1 to 800 mg daily once or several times per day for consecutive days is preferable. When the dose of the compound is within this range, the preventive effect or the therapeutic effect on muscle atrophy and muscle regeneration failure is considered to be exhibited.

The dose of the nitration inhibitor and the preventive or therapeutic agent for muscle atrophy and muscle regeneration failure of the present invention is a therapeutically effective amount and varies depending on symptoms, age, administration route, dosage form, and the like. In typical cases, glutathione trisulfide or a pharmaceutically acceptable salt thereof or Compound (1), a pharmaceutically acceptable salt thereof, or a cyclodextrin clathrate thereof can be administered to a companion animal at 0.1 to 400 mg/kg per day, and the administration of 0.1 to 400 mg/kg daily once or several times per day for consecutive days is preferable. When the dose of the compound is within this range, the preventive effect or the therapeutic effect on muscle atrophy and muscle regeneration failure is considered to be exhibited.

According to another embodiment of the present invention, an agent for suppressing or improving inhibition of muscle growth of livestock or poultry (reduction in meat productivity) caused by heat stress comprises the nitration inhibitor.

Examples of the cause of inhibition of muscle growth of livestock or poultry (decrease in meat productivity) due to heat stress in the present invention include an increase in outside air temperature of livestock or poultry breeding environment due to heat in summer, abnormality or deviation in temperature management in a livestock or poultry breeding management area, and the like. These causes are thought to induce respiratory alkalosis, decreased thyroid hormones, increased corticosterone, altered immune responses and excessive production of mitochondrial reactive oxygen species (ROS) in livestock or poultry. As a result, inhibition of activation or decrease in proliferation of muscle stem cells (satellite cells) causes the disorder of growth, hypertrophy, regeneration or maintenance of muscles, resulting in a decrease in meat productivity.

Examples of livestock or poultry include cattle, horses, pigs, donkeys, sheep, goats, chickens, ducks, geese, ostrich, turkeys, ducks, quail, and the like.

The dose of the nitration inhibitor and the agent for suppressing or improving inhibition of muscle growth of livestock or poultry (reduction in meat productivity) is a therapeutically effective amount and varies depending on symptoms, administration route, and the like. In typical cases, glutathione trisulfide or a pharmaceutically acceptable salt thereof or Compound (1), a pharmaceutically acceptable salt thereof, or a cyclodextrin clathrate thereof can be administered to livestock or poultry at 0.1 to 400 mg/kg per day, and the administration of 0.1 to 400 mg/kg daily once or several times per day for consecutive days is preferable. When the dose of the compound is within this range, the suppressing or improving effect on the inhibition of muscle growth of livestock or poultry (reduction in meat productivity) is considered to be exhibited.

The nitration inhibitor, the preventive or therapeutic agent for muscle atrophy and/or muscle regeneration failure, and the agent for suppressing or improving inhibition of muscle growth (reduction in meat productivity) of the invention can be administered orally, for example, as tablets, capsules, powders, granules, solutions or syrups, or parenterally, for example, as injections, infusions or suppositories. These dosage forms can be formulated by known formulation techniques. In the case of solid preparations, pharmaceutically acceptable excipients such as starch, lactose, white soft sugar, glucose, crystalline cellulose, carboxycellulose, carboxymethylcellulose, carboxyethylcellulose, calcium phosphate, magnesium stearate, gum Arabic and the like can be added in the preparation, and if necessary, lubricants, binders, disintegrants, coating agents, coloring agents and the like can be added. In the case of liquid preparations, stabilizers, dissolution aids, suspending agents, emulsifying agents, buffers, preservatives and the like can be added.

### [Example]

Hereinafter, the present invention will be described in detail with reference to examples, but the present invention is not limited to these examples. GSSSG means glutathione trisulfide.

### EXAMPLE 1

### <Inhibitory effect of GSSSG on nitration of recombinant HGF protein by peroxynitrite (ONOO⁻)>

GSSSG dihydrate produced by the method described in Patent Literature 1 was added to and mixed with a purified preparation of recombinant mouse HGF (2207-HG-025/CF, manufactured by R&D Systems, carrier free) dissolved in PBS such that the molar ratio of HGF:GSSSG was 1:10, 1:50, or 1 :400 to prepare samples. A sample was also prepared in which GSSSG dihydrate was not added and the molar ratio of HGF:GSSSG was 1:0. Peroxynitrite (ONOO⁻) was immediately added to each sample such that the molar ratio of HGF:ONOO⁻ was 1:500. The samples were held at pH 7.2 to 7.4 and 25 to 37 °C for 30 minutes (nitrating treatment) to give nitrated samples.

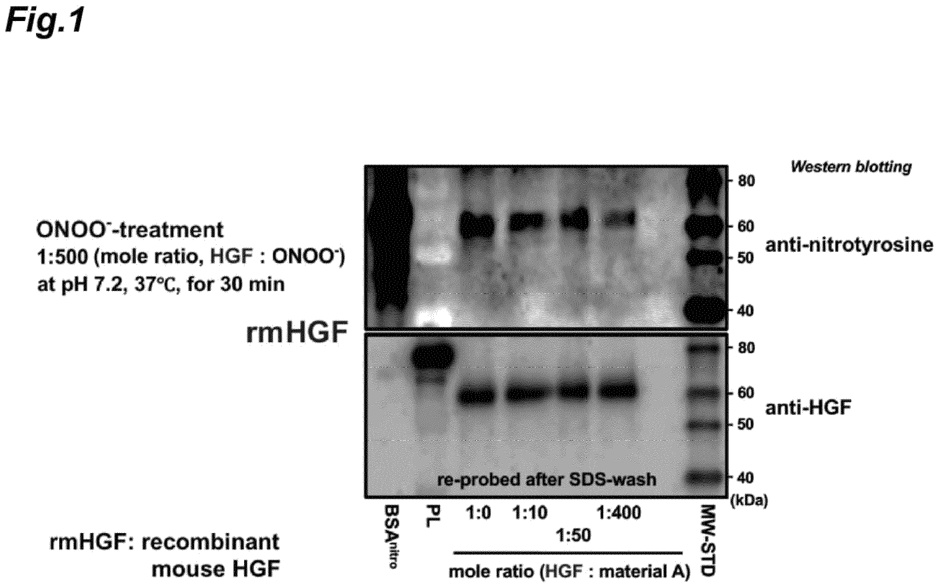

The obtained nitrated samples were subjected to Western blotting in accordance with a conventional method. The nitrated samples were treated with a solution containing SDS and β-mercaptoethanol to give samples for reduced SDS-PAGE. A 10% polyacrylamide gel was used for electrophoresis. The material of the transfer membrane used was nitrocellulose. HRP-labeled anti-nitrotyrosine monoclonal antibody (catalog number: sc-32757 HRP, manufactured by Santa Cruz Biotechnology) and HRP-labeled anti-HGF monoclonal antibody (catalog number: sc-374422 HRP, manufactured by Santa Cruz Biotechnology) were used. The immune response was recorded on an imaging device using ECL reagents. Nitrated bovine serum albumin (BSA^{nitro}) was used as a positive control sample. PL (Protein Ladder) and MW-STD (Molecular Weight-Standard) in Fig. 1 are molecular weight markers. Further, material A in Fig. 1 means GSSSG.

After confirming that the reaction intensity with the anti-HGF antibody was substantially the same in each lane (lower frame in Fig. 1), it was confirmed that the reaction intensity with the anti-nitrotyrosine antibody decreased with an increase in the molar ratio of GSSSG added (upper frame in Fig. 1). That is, the nitration inhibitory effect on tyrosine residues in HGF by GSSSG was visualized.

### Example 2

### <Inhibitory effect of lipoic acid trisulfide on nitration of recombinant HGF protein by peroxynitrite (ONOO')>

Nitration treatment and Western blotting were performed in the same manner as in Example 1 except that lipoic acid trisulfide was used instead of GSSSG and the molar ratio of HGF:ONOO⁻ was set to 1:4000. In Fig. 1, material B means lipoic acid trisulfide.

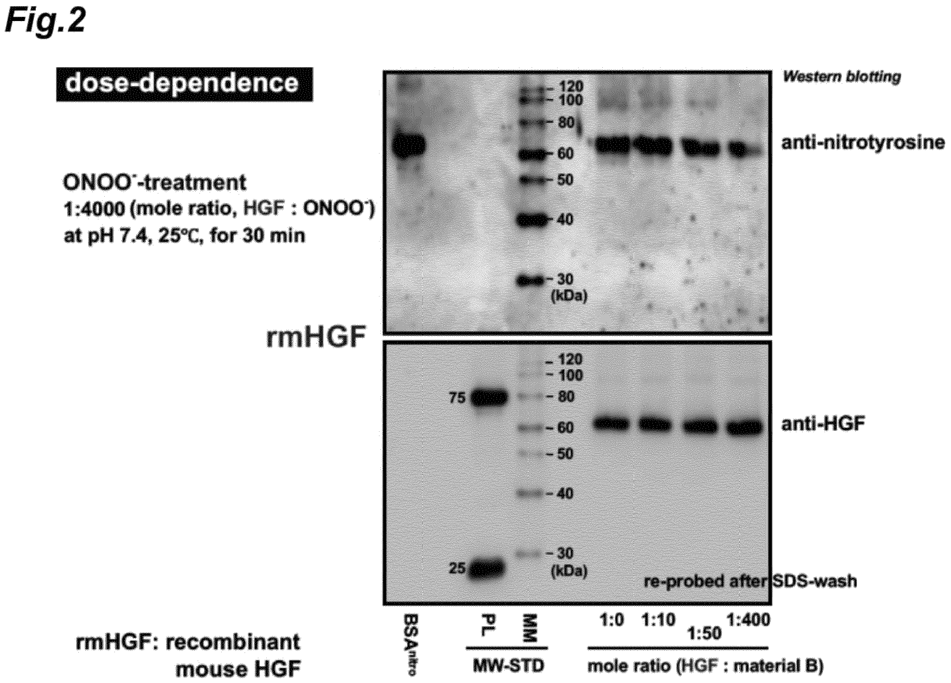

After confirming that the reaction intensity with the anti-HGF antibody was substantially the same in each lane (lower frame in Fig. 2) as in Example 1, it was confirmed that the reaction intensity with the anti-nitrotyrosine antibody decreased with an increase in the molar ratio of lipoic acid trisulfide added (upper frame in Fig. 2). That is, the nitration inhibitory effect on tyrosine residues in HGF by lipoic acid trisulfide was visualized.

Since GSSSG or a pharmaceutically acceptable salt thereof or compound (1), a pharmaceutically acceptable salt thereof or a cyclodextrin clathrate thereof is considered to be capable of inhibiting nitration of tyrosine residues in HGF by peroxynitrite (ONOO⁻), they are expected to also inhibit dysfunction of HGF caused by various oxidative stresses occurring *in vivo.* GSSSG or a pharmaceutically acceptable salt thereof or compound (1), a pharmaceutically acceptable salt thereof or a cyclodextrin clathrate thereof is considered to exert a preventive or therapeutic effect on muscle atrophy and muscle regeneration failure caused by nitration of tyrosine residues in HGF. GSSSG or a pharmaceutically acceptable salt thereof, or compound (1), a pharmaceutically acceptable salt thereof, or a cyclodextrin clathrate thereof is considered to be capable of suppress or improve inhibition of muscle growth (decrease in meat productivity) caused by nitration of tyrosine residues in HGF due to heat stress.

### REFERENCE EXAMPLE 1

### <Production of (R)-lipoic acid trisulfide>

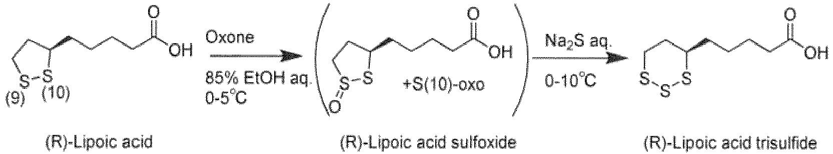

24.38 g (118.17 mmol) of (R)-α-lipoic acid and 488 mL (20.0 v/w) of a 75% ethanol aqueous solution were added to a 200 mL four-neck flask. After confirming that the contents of the flask had dissolved, the internal temperature was cooled to 0°C. Oxone (registered trademark) (41.40 g, 124.20 mmol, 1.05 equivalents) was added thereto in two portions to cause a reaction for about 50 minutes. An insoluble substance in the reaction solution was removed by filtration and then washed with 65 mL (2.67 v/w) of ethanol. 400 mL (206.93 mmol, 1.75 equivalents) of a Na₂S aqueous solution (70.70 g of Na₂S·9H₂O dissolved in 569 mL of water) was added dropwise to the filtrate and wash liquid at an internal temperature of 2°C to 6°C over about 2.5 hours (during the dropwise addition and the reaction, a 3 mol/L sulfuric acid aqueous solution was used to control the pH at 6 to 7, and the total amount thereof used was 14 mL). After causing a reaction at an internal temperature of 3°C at a pH 7 for about 50 minutes, 41 mL (1.7 v/w) of a 3 mol/L sulfuric acid aqueous solution was added dropwise thereto to adjust the pH to 1.3. Next, 320 mL (13.1 v/w) of water and 320 mL (13.1 v/w) of ethyl acetate were added thereto to perform extraction with ethyl acetate. The aqueous layer was extracted four times with 160 mL (6.6 v/w) of ethyl acetate, and organic layers were combined and concentrated under reduced pressure at an external temperature of 30°C. Ethanol was added to the concentrate to dissolve it, followed by column purification with ODS. The fraction was concentrated under reduced pressure at an external temperature of 30°C and then dried with an oil pump to obtain 10.69 g of (R)-lipoic acid trisulfide (44.84 mmol, yield 38%, HPLC purity of 99.7%, white solid).

### <Production of (R)-lipoamide trisulfide>

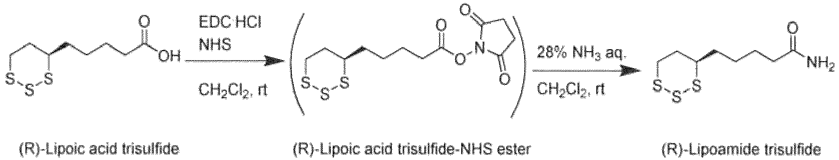

2.00 g (8.39 mmol) of (R)-lipoic acid trisulfide and 65 mL (32.5 v/w) of methylene chloride were added to a 200 mL four-neck flask. After confinning that the contents of the flask had dissolved, 2.07 g (10.77 mmol, 1.28 equivalents) of 1-(3-dimethylaminopropyl)-3-ethylcarbodiimide hydrochloride (EDC·HCl) and 1.42 g (12.33 mmol, 1.47 equivalents) of N-hydroxysuccinimide (NHS) were added thereto. After replacing the air in the flask with nitrogen, a reaction was carried out at room temperature for about 8 hours. Next, 2.28 mL (33.74 mmol, 4.02 equivalents) of 28%) aqueous ammonia was added dropwise at room temperature over 5 minutes and allowed to react overnight. Thereafter, at room temperature, 60 mL of water (30.0 v/w) was added thereto and the mixture was subjected to liquid separation. Then, the organic layer was washed three times with 60 mL (30.0 v/w) of a 2.5% sodium hydrogen carbonate aqueous solution and additionally washed four times with 60 mL of water (30.0 v/w). Thereafter, the organic layer after washing was concentrated under reduced pressure at an external temperature of 25°C, and then dried with an oil pump to obtain 1.93 g of (R)-lipoamide trisulfide (8.13 mmol, yield 97%, HPLC purity of 99.6%, white solid).
¹H-NMR: (CDCl₃, 400 MHz) δ (ppm)=5.36 (bs, 2H), 3.33 (m, 1H), 3.13 (m, 2H), 2.22 (m, 3H), 1.89 (m, 1H), 1.74-1.42 (m, 6H). HR-ESI-TOF-MS: m/z 236.0238 ([M-H]⁻), calcd for [C₈H₁₄NOS₃]-236.0243.

### Reference Example 2

### <Production of lipoamide trisulfide (racemate)>

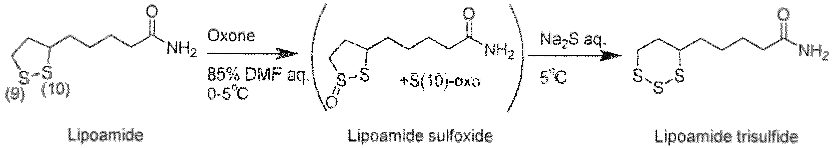

1.00 g (4.87 mmol) of lipoamide (racemate) and 182 mL (182.0 v/w) of an 85% dimethylformamide aqueous solution were added to a 500 mL four-neck flask. After confirming that the contents of the flask had dissolved, the internal temperature was cooled to 4°C. 1.63 g (4.89 mmol, 1.00 equivalent) of Oxone (registered trademark) was added to the flask in three portions every 10 minutes to cause a reaction for about 1 hour. 1.24 g (5.16 mmol, 1.06 equivalents) of sodium sulfide nonahydrate was added in portions at an internal temperature of 5°C while controlling the pH of the reaction solution at 5 to 11 using a 3 mol/L sulfuric acid aqueous solution to cause a reaction for about 1.5 hours. 180 mL (180.0 v/w) of water and 50 mL (50.0 v/w) of methylene chloride were added thereto to perform extraction with methylene chloride. Then, the aqueous layer was extracted twice with 50 mL (50.0 v/w) of methylene chloride, and organic layers were combined and concentrated under reduced pressure at an external temperature of 30°C or lower. 80 mL (80.0 v/w) of water was added dropwise to the concentrated residue over 30 minutes at room temperature for crystallization, and the slurry was filtered and then washed with 50 mL (50.0 v/w) of water. Wet crystals were dried under reduced pressure at 25°C to obtain 510 mg of lipoamide trisulfide (racemate) (2.15 mmol, yield 44%, HPLC purity of 92%, white solid).

### <Purity test (HPLC) of lipoamide trisulfide>

Detector: Ultraviolet absorptiometer (measurement wavelength: 220 nm)
Column: LiChrosorb RP-18 (Kanto Chemical Co., Inc., 4.0 mm I.D. × 250 mm, 5 µm)
Column temperature: Constant temperature around 40°C
Mobile phase A: Phosphoric acid aqueous solution (pH 3)
Mobile phase B: Methanol
Mobile phase delivery: The mixing ratio of the mobile phase A and the mobile phase B was changed as follows to control the concentration gradient.

**Table 1**

| Time after injection (minute) | Mobile phase A (vol%) | Mobile phase B (vol%) |
|---|---|---|
| 0 to 5 | 100 | 0 |
| 5 to 15 | 100 → 25 | 0 → 75 |
| 15 to 20 | 25 | 75 |
| 20 to 21 | 25 → 100 | 75 → 0 |
| 21 to 35 | 100 | 0 |

Flow rate: 1 mL/min
Injection volume: 10 µL
Area measurement range: 35 minutes after injecting sample solution
Holding time: lipoamide sulfoxide (12 to 13 minutes), lipoamide (about 17 minutes), and lipoamide trisulfide (about 19 minutes)

### Reference Examples 3 to 9

Hereinafter, "HP", "Me", and "Ma1" are respectively abbreviations for "hydroxypropyl", "methyl", and "maltosyl."

### <Production of lipoic acid trisulfide>

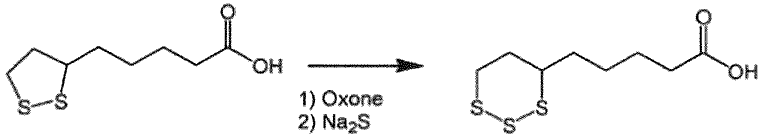

2.0 g (9.02 mmol) of lipoic acid and 40 mL of a 75% ethanol aqueous solution were added to a reaction container, and the mixture was cooled to an internal temperature of 0°C. 3.4 g (10.20 mmol) of Oxone (registered trademark) was added thereto to cause a reaction for about 2 hours. Inorganic salts in the reaction solution were filtered and then washed in 7 mL of ethanol. 5.8 g (24.1 mmol) of sodium sulfide nonahydrate was added to the filtrate to cause a reaction for about 1 hour. After 7 mL of a 3 mol/L sulfuric acid aqueous solution was added dropwise to this reaction solution, 20 mL of water and 45 mL of ethyl acetate (AcOEt) were subsequently added thereto to perform extraction with AcOEt. The aqueous layer was extracted twice with 20 mL of AcOEt, and organic layers were combined and concentrated under reduced pressure. After 3 mL of ethanol was added to the concentrate to dissolve it, the solution was purified with an CDS column (YMC Dispo PackAT, mobile phase: acetonitrile aqueous solution) to obtain 0.7 g (2.39 mmol, HPLC purity: 100%) of lipoic acid trisulfide.

### <Production of CD clathrate of lipoic acid trisulfide>

### Reference Example 3: β-CD clathrate of lipoic acid trisulfide (racemate)

1,020.0 mg (0.899 mmol) of β-CD and 80 mL of water were added to a 100 mL eggplant flask. After confirming that the contents of the flask had dissolved, 99.8 mg (0.419 mmol) of lipoic acid trisulfide was added thereto, and the inside of the flask was washed with 20 mL of water. The washed mixture was stirred at 45°C for 15 minutes and then filtered, and the inside of the flask and the crystals were washed with 10 mL of water. The obtained filtrate was frozen in a -20°C freezer for 23 hours. The frozen filtrate was freeze-dried at an external temperature of 20°C for about 4.5 days to obtain 980.0 mg of a clathrate (white solid).

### Reference Example 4: HP-β-CD clathrate of lipoic acid trisulfide (racemate)

1291.0 mg of HP-β-CD and 16 mL of water were added to a 50 mL eggplant flask. After confirming that the contents of the flask had dissolved, 100.0 mg (0.419 mmol) of lipoic acid trisulfide was added thereto. The mixture was stirred at room temperature for about 28 hours and then filtered, and the inside of the flask and the crystals were washed with 10 mL of water. The obtained filtrate was frozen in a -20°C freezer for about 2 days. The frozen filtrate was freeze-dried at an external temperature of 20°C for about 2 days to obtain 1330.0 mg of a clathrate (white solid).

### Reference Example 5: HP-β-CD clathrate of (R)-lipoic acid trisulfide

969.9 mg of HP-β-CD and 10 mL of water were added to a 50 mL eggplant flask. After confirming that the contents of the flask had dissolved, 100.3 mg (0.421 mmol) of (R)-lipoic acid trisulfide was added thereto, and the inside of the flask was washed with 4 mL of water. The washed mixture was stirred at room temperature for about 25 hours and then filtered, and the inside of the flask and the crystals were washed with 12 mL of water. The obtained filtrate was frozen in a -20°C freezer for 15 hours. The frozen filtrate was freeze-dried at an external temperature of 20°C for about 2 days to obtain 1040.0 mg of a clathrate (white solid).

### Reference Example 6: Me-P-CD clathrate of lipoic acid trisulfide (racemate)

1,616.0 mg of Me-p-CD (mixture of several methylated) and 12 mL of water were added to a 50 mL eggplant flask. After confirming that the contents of the flask had dissolved, 101.0 mg (0.424 mmol) of lipoic acid trisulfide was added thereto, and the inside of the flask was washed with 4 mL of water. The washed mixture was stirred for 21 hours and then filtered, and the inside of the flask and the crystals were washed with 12 mL of water. The obtained filtrate was frozen in a -20°C freezer for 20 hours. The frozen filtrate was freeze-dried at an external temperature of 20°C for about 4 days to obtain 1665.2 mg of a clathrate (white solid).

### Reference Example 7: ME-0-CD clathrate of (R)-lipoic acid trisulfide

1,616.0 mg of Me-p-CD (mixture of several methylated) and 16 mL of water were added to a 50 mL eggplant flask. After confirming that the contents of the flask had dissolved, 99.9 mg (0.420 mmol) of (R)-lipoic acid trisulfide was added thereto, and the inside of the flask was washed with 4 mL of water. The washed mixture was stirred at room temperature for about 6 hours and then filtered, and the inside of the flask and the crystals were washed with 13 mL of water. The obtained filtrate was frozen in a -20°C freezer for 28 hours. The frozen filtrate was freeze-dried at an external temperature of 20°C for about 3 days to obtain 1610.9 mg of a clathrate (white solid).

### Reference Example 8: Mal-β-CD clathrate of lipoic acid trisulfide (racemate)

1,224.2 mg (0.839 mmol) of Mal-β-CD and 14 mL of water were added to a 50 mL eggplant flask. After confirming that the contents of the flask had dissolved, 100.4 mg (0.421 mmol) of lipoic acid trisulfide was added thereto, and the inside of the flask was washed with 2 mL of water. The washed mixture was stirred at room temperature for about 31 hours and then filtered, and the inside of the flask and the crystals were washed with 10 mL of water. The obtained filtrate was frozen in a -20°C freezer for 22 hours. The frozen filtrate was freeze-dried at an external temperature of 20°C for about 46 hours to obtain 1180.0 mg of a clathrate (white solid).

### Reference Example 9: Mal-β-CD clathrate of (R)-lipoic acid trisulfide

1,224.2 mg (0.839 mmol) of Mal-β-CD and 10 mL of water were added to a 50 mL eggplant flask. After confirming that the contents of the flask had dissolved, 100.1 mg (0.420 mmol) of (R)-lipoic acid trisulfide was added thereto, and the inside of the flask was washed with 5 mL of water. The washed mixture was stirred at room temperature for about 4.5 hours and then filtered, and the inside of the flask and the crystals were washed with 11 mL of water. The obtained filtrate was frozen in a -20°C freezer for 24 hours. The frozen filtrate was freeze-dried at an external temperature of 20°C for about 41 hours to obtain 1319.6 mg of a clathrate (white solid).

The yield and solubility of the clathrates obtained in Reference Examples 3 to 9 are shown in Table 2

**[Table 2]**

| E x | Lipoic acid trisulfide | | β-CD | | Clathrate (freeze-dried product) | | | |
|---|---|---|---|---|---|---|---|---|
| | Type | Solubili ty (g/L¹⁾) | Modi ficati on | Charge amount (w/w) | Percent yield (%)²⁾ | Content (%) | | Solubilit y (g/L³⁾) |
| | | | | | | Actual measurement | Theoretic al value | |
| 3 | Racemate | 0.1 | None | 10.2 | 84 | 8.6 | 8.9 | 0.77 |
| 4 | Racemate | 0.1 | HP | 12.9 | 94 | 7.1 | 7.2 | ≥49 |
| 5 | R body | 0.3 | | 9.7 | 99 | 9.6 | 9.4 | ≥65 |
| 6 | Racemate | 0.1 | Me | 16.0 | 96 | 5.8 | 5.9 | ≥50 |
| 7 | R body | 0.3 | | 16.2 | 99 | 6.1 | 5.8 | ≥41 |
| 8 | Racemate | 0.1 | Mal | 12.2 | 88 | 7.5 | 7.6 | ≥45 |
| 9 | R body | 0.3 | | | 105 | 8.0 | 7.6 | ≥52 |

| | | | | | | | | |
|---|---|---|---|---|---|---|---|---|
| ¹⁾ represents solubility in water at 20°C. ²⁾ Percent yield (%) = (yield × content)/theoretical yield × 100 ³⁾ represents solubility of lipoic acid trisulfide in a clathrate in water at 20°C. A statement such as "≥49 g/L" indicates dissolution at 49 g/L. | | | | | | | | |

## Claims

1. A nitration inhibitor of a tyrosine residue in hepatocyte growth factor, comprising a trisulfide compound,
wherein the trisulfide compound is glutathione trisulfide or a pharmaceutically acceptable salt thereof or a compound of formula (1): 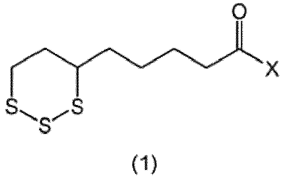 wherein X represents -OR¹ or -NR²R³, R¹ represents a hydrogen atom or an alkyl group having 1 to 6 carbon atoms, R² and R³ each independently represent a hydrogen atom or an alkyl group having 1 to 6 carbon atoms, and the alkyl group optionally has one or more substituents selected from the group consisting of an amino group and a carboxy group, or a pharmaceutically acceptable salt thereof or a cyclodextrin clathrate thereof.

2. The nitration inhibitor according to claim 1, wherein the trisulfide compound is glutathione trisulfide or a pharmaceutically acceptable salt thereof.

3. The nitration inhibitor according to claim 1, wherein the trisulfide compound is lipoic acid trisulfide or a pharmaceutically acceptable salt thereof.

4. A preventive or therapeutic agent for muscle atrophy and/or muscle regeneration failure, comprising the nitration inhibitor according to any one of claims 1 to 3.

5. A method for preventing or treating muscle atrophy and/or muscle regeneration failure in a companion animal, comprising administering the nitration inhibitor according to any one of claims 1 to 3 to the companion animal.

6. An agent for suppressing or improving inhibition of muscle growth of livestock or poultry (decrease in meat productivity) caused by heat stress, comprising the nitration inhibitor according to any one of claims 1 to 3.

7. A method for suppressing or improving inhibition of muscle growth of livestock or poultry (decrease in meat productivity) caused by heat stress, comprising administering the nitration inhibitor according to any one of claims 1 to 3 to the livestock or the poultry.
